# EUROPEAN PATENT SPECIFICATION

(11) **EP 2 635 256 B1**
(45) Date of publication and mention of the grant of the patent: **21.10.2020**
(21) Application number: 11838922.0
(22) Date of filing: 04.11.2011
(51) Int. Cl.: A61K 31/44, A61K 31/198, A61K 9/00

(54) **A2A ANTAGONISTS AS COGNITION AND MOTOR FUNCTION ENHANCERS**
A2A ANTAGONISTEN ALS VERSTÄRKER VON KOGNITIVEN UND MOTORISCHEN FUNKTIONEN
ANTAGONISTES A2A EN TANT QUE STIMULATEURS DE LA COGNITION ET DE LA FONCTION MOTRICE

(30) Priority: 05.11.2010 WO PCT/US2010/055681
(43) Date of publication of application: 11.09.2013
(73) Proprietor: Biotie Therapies, Inc., South San Francisco, CA 94080 (US)
(72) Inventor: BANDAK, Stephen, I., CA 94080 (US); BLACK, Kevin, J., CA 94080 (US); CAMPBELL, Meghan, C., CA 94080 (US)
(74) Representative: Goodfellow, Hugh Robin
(86) International application number: PCT/US2011/059466
(87) International publication number: WO 2012/061787

(56) References cited:
- WO-A1-2005/116026
- WO-A2-2010/148121
- US-A1- 2005 261 289
- US-A1- 2006 178 379
- Anonymous: "Synosia's SYN-115 demonstrates clinical benefit in Parkinson's disease", Internet , 14 April 2010 (2010-04-14), XP002722791, Retrieved from the Internet: URL:www.drugs.com/clinical_trials [retrieved on 2014-04-01]
- Anonymous: "Synosia therapeutics' SYN-115 improves motor and non-motor function in patients with mild-to-moderate Parkinson's disease", Internet , 14 April 2010 (2010-04-14), XP002722792, Retrieved from the Internet: URL:www.businesswire.com/news/home [retrieved on 2014-04-01]
- Anonymous: "SYN-115", Internet , 16 December 2005 (2005-12-16), XP002722793, Retrieved from the Internet: URL:https://integrity.thompson-pharma.com [retrieved on 2014-04-02]
- BLACK KEVIN J ET AL: "A Randomized, Double-Blind, Placebo-Controlled Cross-Over Trial of the Adenosine 2a Antagonist SYN115 in Parkinson Disease", NEUROLOGY, vol. 74, no. 9, Suppl. 2, March 2010 (2010-03), page A317, XP009177333, & 62ND ANNUAL MEETING OF THE AMERICAN-ACADEMY-OF-NEUROLOGY; TORONTO, CANADA; APRIL 10 -17, 2010 ISSN: 0028-3878
- DICKERSON W ET AL: "The adenosine 2a antagonist SYN115 decreases thalamic rCBF in Parkinson disease", SOCIETY FOR NEUROSCIENCE ABSTRACT VIEWER AND ITINERARY PLANNER, vol. 39, 2009, XP002722795, & 39TH ANNUAL MEETING OF THE SOCIETY-FOR-NEUROSCIENCE; CHICAGO, IL, USA; OCTOBER 17 -21, 2009
- REZAK ET AL: "Current Pharmacotherapeutic Treatment Options in Parkinson's Disease", DISEASE-A-MONTH, YEAR BOOK PUBL., CHICAGO, IL, US, vol. 53, no. 4, 1 April 2007 (2007-04-01), pages 214-222, XP022128103, ISSN: 0011-5029, DOI: 10.1016/J.DISAMONTH.2007.05.002
- SANCHEZ-CASTANEDA C ET AL: "Cognitive improvement after duodenal levodopa infusion in cognitively impaired Parkinson's disease patients", PROGRESS IN NEURO-PSYCHOPHARMACOLOGY & BIOLOGICAL PSYCHIATRY, ELSEVIER, GB, vol. 34, no. 1, 1 February 2010 (2010-02-01), pages 250-251, XP026832729, ISSN: 0278-5846 [retrieved on 2009-11-01]
- LEIVA-SANTANA C ET AL: "[Levodopa and cognitive disorders in Parkinson's disease].", REVISTA DE NEUROLOGIA 2006 JUL 16-31, vol. 43, no. 2, 16 July 2006 (2006-07-16), pages 95-100, XP009189851, ISSN: 0210-0010

## Description

### FIELD

The present disclosure relates to methods and pharmaceutical compositions for the treatment of symptoms associated with a neurodegenerative disease such as Parkinson's disease. In particular, the present disclosure relates to 4-hydroxy-4-methyl-piperidine-1-carboxylic acid-(4-methoxy-7-morpholin-4-yl-benzothiazol-2-yl)-amide and to pharmaceutically acceptable salts thereof, and their use in the treatment of Parkinson's disease and other related diseases. The invention is limited to the subject-matter defined in the appended claims; the following description is limited to this limitation.

### BACKGROUND

Parkinson's disease is a disabling, progressive illness that generally occurs in people over the age of 50 years. The incidence of Parkinson's disease increases with age and the cumulative lifetime risk of an individual developing the disease is approximately 1 in 40. While Parkinson's disease is primarily defined by its motor symptoms - including pronounced tremor of the extremities, bradykinesia, rigidity and postural changes - there are also significant behavioral alterations that can severely impact patient quality-of-life (Jankovic, 2008, J Neurol Neurosurg Psychiatry 79 368-376). The non-motor symptoms of Parkinson's disease include sleep disorders, depression, anxiety, psychosis and cognitive decline. Patients with Parkinson's disease have a deficiency of the neurotransmitter dopamine in the brain. Degeneration of the substantia nigra and subsequent disruption of the nigrostriatal pathway has been linked to motor symptoms while loss of dopamine in the basal ganglia, with degeneration in the striato-nigral regions, has been associated with cognitive decline and other non-motor symptoms. It is likely that several other neurotransmitter systems are also disrupted in the frontal lobes contributing to the overall range of cognitive impairments described in Parkinson's disease.

Levodopa (L-dopa or L-3,4-dihydroxyphenylalanine), an immediate precursor of dopamine, is the most commonly prescribed drug for treatment of Parkinson's disease. The bioavailability of levodopa is dose-dependent due to saturation of the active transport pathway. Plasma levels of levodopa must be carefully titrated for each patient to achieve the optimal therapeutic activity. If the concentration of levodopa is too low in plasma (and consequently in the brain) the patient can experience a return of the symptoms of Parkinson's disease (e.g., rigidity, tremor, and bradykinesia). On the other hand, motor fluctuation can become a significant side effect if plasma drug levels are too high. Uncontrolled fluctuations in plasma levodopa levels can greatly contribute to the incidence of "on-off' fluctuations (dyskinesias).

In addition, levodopa is rapidly decarboxylated to dopamine by L-aromatic amino acid decarboxylase (AADC) in the peripheral tissues. The intestinal metabolism of levodopa is the major source of first pass loss of the drug, and only 1% of the administered dose is able to transport across the blood-brain barrier. For this reason, levodopa is normally co-administered with a drug designed to inhibit its peripheral decarboxylation, such as carbidopa or benserazide. Carbidopa and benserazide themselves do not cross the blood-brain barrier to a significant extent, and therefore do not inhibit the required conversion of levodopa to dopamine in the brain.

There remains a continuing need for new therapies for the treatment of patients having Parkinson's disease and related diseases, including the symptoms associated with such neurodegenerative diseases. In particular, the treatment of non-motor symptoms, which can affect >50% of Parkinson's disease patients, is now increasingly recognized as an area of high unmet medical need. The present disclosure fulfills these needs.

### SUMMARY

The present disclosure provides compounds that are A2a antagonists, compositions comprising the compounds, and methods of using the compounds, including in the treatment and/or prevention of diseases mediated by the adenosine receptor. In particular, the present disclosure provides methods and compositions for the treatment of symptoms associated with neurodegenerative diseases by administering an A2a antagonist optionally in combination with a dopamine precursor or dopamine receptor agonist.

Accordingly, in one aspect, the present disclosure provides methods of inhibiting the adenosine receptor comprising contacting an adenosine receptor with an effective amount of a compound or composition of the present disclosure effective for inhibition. The methods can be practiced either *in vitro* or *in vivo,* and can be used as a therapeutic approach towards the treatment and/or prevention of diseases associated with the adenosine receptor.

In another aspect, the present disclosure relates to a method for treating the symptoms associated with a neurodegenerative disease in a patient. The method of the present disclosure comprises concomitantly administering to the patient a therapeutically effective amount of at least one A2a antagonist with a therapeutically effective amount of a dopamine precursor or a therapeutically effective amount of a dopamine receptor agonist. The A2a antagonist can be 4-hydroxy-4-methyl-piperidine-1-carboxylic acid-(4-methoxy-7-morpholin-4-yl-benzothiazol-2-yl)-amide, the dopamine precursor can be levodopa, and the dopamine receptor agonist can be apomorphine, pramipexole, bromocriptine, cabergoline, ropinirole, or rotigotine.

The present disclosure also relates to a pharmaceutical composition for treating of the symptoms associated with a neurodegenerative disease and to a composition for use in the treatment of symptoms associated to a neurodegenerative disease. Compositions comprise a therapeutically effective amount of 4-hydroxy-4-methyl-piperidine-1-carboxylic acid-(4-methoxy-7-morpholin-4-yl-benzothiazol-2-yl)-amide with a therapeutically effective amount of a dopamine precursor or a therapeutically effective amount of a dopamine receptor agonist.

The present disclosure further relates to the use of a composition in the manufacture of a medicament for the treatment of symptoms associated with a neurodegenerative disease, where the composition comprises a therapeutically effective amount of 4-hydroxy-4-methyl-piperidine-1-carboxylic acid-(4-methoxy-7-morpholin-4-yl-benzothiazol-2-yl)-amide with a therapeutically effective amount of a dopamine precursor or a therapeutically effective amount of a dopamine receptor agonist.

The present disclosure also relates to the use of a composition for the treatment of symptoms associated with a neurodegenerative disease, where the composition comprises a therapeutically effective amount of 4-hydroxy-4-methyl-piperidine-1-carboxylic acid-(4-methoxy-7-morpholin-4-yl-benzothiazol-2-yl)-amide with a therapeutically effective amount of a dopamine precursor or a therapeutically effective amount of a dopamine receptor agonist.

Also provided are methods of treating at least one symptom associated with Parkinson's disease, in which the at least one symptom can be cognition impairment or decline, a motor symptom such as tremors, muscle stiffness, joint stiffness, spasm, low muscle control, movement difficulty, rigidity in arms, rigidity in legs, reduced locomotor activity, and movement coordination, or any combination thereof. The methods include administering to the patient a therapeutically effective amount of 4-hydroxy-4-methylpiperidine-1-carboxylic acid-(4-methoxy-7-morpholin-4-yl-benzothiazol-2-yl)-amide, optionally in combination with a dopamine precursor, such as levodopa, or a dopamine receptor agonist.

Also provided are methods of treating at least one symptom associated with Parkinsonism, in which the at least one symptom can be cognition impairment or decline, a motor symptom such as tremors, muscle stiffness, joint stiffness, spasm, low muscle control, movement difficulty, ipsilateral circling, rigidity in arms, rigidity in legs, reduced locomotor activity, and movement coordination, or any combination thereof. The methods include administering to the patient a therapeutically effective amount of 4-hydroxy-4-methylpiperidine-1-carboxylic acid-(4-methoxy-7-morpholin-4-yl-benzothiazol-2-yl)-amide, optionally in combination with a dopamine precursor, such as levodopa, or a dopamine receptor agonist.

### BRIEF DESCRIPTION OF THE FIGURES

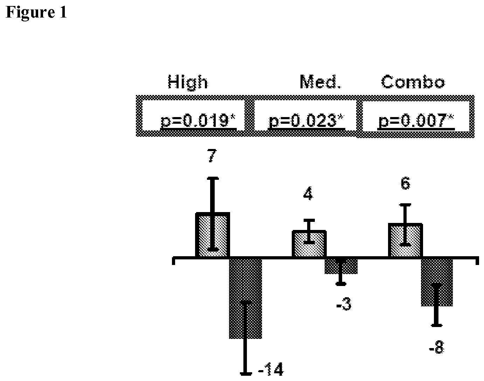

**Figure 1** illustrates the effect of the A2a antagonist (dark-colored bars) compared with the placebo (light-colored bars) on the Go/No-Go task reaction time and shows that the reaction times are faster with fewer mistakes for patients treated with 4-hydroxy-4-methylpiperidine-1-carboxylic acid(4-methoxy-7-morpholin-4-yl-benzothiazol-2-yl)-amide.

### DETAILED DESCRIPTION

The descriptions of various embodiments of the invention are presented for purposes of illustration, and are not intended to be exhaustive or to limit the invention to the forms disclosed. Persons skilled in the relevant art can appreciate that many modifications and variations are possible in light of the embodiment teachings.

Unless otherwise stated, the following terms used in this application, including the specification and claims, have the definitions given below. It must be noted that, as used in the specification and the appended claims, the singular forms "a," "an" and "the" include plural referents unless the context clearly dictates otherwise. The definition of standard chemistry terms can be found in reference works, including Carey and Sundberg (1992) "Advanced Organic Chemistry 3rd Ed." Vols. A and B, Plenum Press, New York. The practice of the present disclosure will employ, unless otherwise indicated, conventional methods of mass spectroscopy, protein chemistry, biochemistry, recombinant DNA techniques and pharmacology, all of which are within the skill of those in the art.

The term "pharmaceutically acceptable acid addition salts" includes salts with inorganic and organic acids, such as hydrochloric acid, nitric acid, sulfuric acid, phosphoric acid, citric acid, formic acid, fumaric acid, maleic acid, acetic acid, succinic acid, tartaric acid, methane-sulfonic acid, p-toluenesulfonic acid, and the like.

The term "pharmaceutically acceptable vehicle" refers to a diluent, adjuvant, excipient or carrier with which a compound of the present disclosure is administered.

The term "protecting group" refers to a group of atoms that, when attached to a reactive functional group in a molecule, mask, reduce or prevent the reactivity of the functional group. Typically, a protecting group can be selectively removed as desired during the course of a synthesis. Examples of protecting groups can be found in Greene and Wuts, Protective Groups in Organic Chemistry, 3rd Ed., 1999, John Wiley & Sons, NY and Harrison et al., Compendium of Synthetic Organic Methods, Vols. 1-8, 1971-1996, John Wiley & Sons, NY. Representative amino protecting groups include, but are not limited to, formyl, acetyl, trifluoroacetyl, benzyl, benzyloxycarbonyl ("CBZ"), *tert*-butoxycarbonyl ("Boc"), trimethylsilyl ("TMS"), 2-trimethylsilyl-ethanesulfonyl ("SES"), trityl and substituted trityl groups, allyloxycarbonyl, 9-fluorenylmethyloxycarbonyl ("FMOC"), nitro-veratryloxycarbonyl ("NVOC") and the like. Representative hydroxyl protecting groups include, but are not limited to, those where the hydroxyl group is either acylated (*e.g.*, methyl and ethyl esters, acetate or propionate groups or glycol esters) or alkylated such as benzyl and trityl ethers, as well as alkyl ethers, tetrahydropyranyl ethers, trialkylsilyl ethers (*e.g.,* TMS or TIPPS groups) and allyl ethers.

As used herein, the terms "subject" and "patient" are used interchangeably, and each encompasses mammals and non-mammals. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. The terms "subject" and "patient" do not denote a particular age or gender.

As used herein, the terms "treat" or "treatment" are used interchangeably and refer to either (i) a postponement of development of a disease and/or a reduction in the severity of such symptoms that will or are expected to develop or (ii) ameliorating existing symptoms, preventing additional symptoms, and ameliorating or preventing the underlying metabolic causes of symptoms. Treatment can be prophylactic (to prevent or delay the onset of the disease, or to prevent the manifestation of clinical or subclinical symptoms thereof) or therapeutic suppression or alleviation of symptoms after the manifestation of the disease.

Dopamine (D2) receptors co-localize with Adenosine 2a (A2a) receptors in the striatum and thalamus. Activation of the A2a receptor by endogenous adenosine results in a down-regulation of the activity of the D2 receptor and a reduction in motor function. Antagonists of the A2a receptor prevent this down-regulation thereby enhancing the activity of dopamine on the D2 receptor and promoting motor function.

Adenosine antagonists also stimulate the activity of the central nervous system (CNS) and have proven to be effective as cognition enhancers. Shen & Chen, 2009, Current Neuropharmacology 7 195-206. Selective A2a-antagonists have therapeutic potential in the treatment of various forms of dementia, for example in Alzheimer's disease and are useful as neuroprotective agents.

The compounds of the present disclosure can be used to inhibit or reduce the activity of A2a receptor. In these contexts, inhibition and reduction of activity of A2a receptor refers to a lower level of the measured activity relative to a control experiment in which the cells or the subjects are not treated with the test compound. In particular aspects, the inhibition or reduction in the measured activity is at least a 10% reduction or inhibition. One of skill in the art will appreciate that reduction or inhibition of the measured activity of at least 20%, 50%, 75%, 90% or 100%, or any number in between, can be preferred for particular applications.

The present disclosure provides methods for treating or alleviating the symptoms associated with a neurodegenerative disease. The neurodegenerative disease of the present disclosure can be any of disease, disorder, condition, sickness or illness that causes any degeneration, lesion, damage, deterioration or collapsing of neurons, such as dopamine-producing neurons. The neurodegenerative disease includes any of Parkinson's disease (PD), Alzheimer's Disease (AD), Lewy body variant Alzheimer's Disease, amyotrophic lateral sclerosis, dementia, multiple system atrophy, neuronal intranuclear inclusion disease, and Tourettes syndrome. In certain aspects of the present disclosure, the disease is Parkinson's disease.

By treating the symptoms of the neurodegenerative disease, it is intended to mean any way for alleviating, relieving, curing, healing, reducing or improving the symptoms associated with the neurodegenerative disease, and should not be restricted to total or complete disappearance of the symptoms. For the purpose of the present disclosure, symptoms are those generally associated with neurodegenerative diseases and include cognition impairment or decline, tremors, muscle stiffness, joint stiffness, spasm, low muscle control, movement difficulty, ipsilateral circling, rigidity in arms, rigidity in legs, reduced locomotor activity, movement coordination or a combination of these symptoms. For example, use of the method and pharmaceutical composition of the present disclosure supports the notion that 4-hydroxy-4-methyl-piperidine-1-carboxylic acid-(4-methoxy-7-morpholin-4-yl-benzothiazol-2-yl)-amide significantly improves the levodopa-induced locomotor activity.

In one aspect of the present disclosure, the methods and pharmaceutical compositions of the present disclosure comprise an A2a antagonist for the enhancement of cognition in patients suffering from Parkinson's disease. The A2a antagonist can be any one known in the art, such as, for example, 4-hydroxy-4-methyl-piperidine-1-carboxylic acid-(4-methoxy-7-morpholin-4-yl-benzothiazol-2-yl)-amide, as disclosed in U.S. Patent No. 7,368,446 to Flohr et al.

The dopamine precursor used for the purpose of the present disclosure can in various aspects be levodopa, also commonly known as, L-3,4-dihydrophenylalanine, L-dopa or any derivative thereof. Such levodopa derivative includes levodopa methyl ester (LDME, as described in U.S. Patent No. 4,826,875) or L-meta-tyrosine (as described in U.S. Patent No. 3,838,008), levodopa ethyl ester (LDEE, as described in U.S. Patent No. 6,696,600) or salts thereof. Levodopa derivative salts include, but are not limited to, the following: fumarate salt, fumarate dihydrate salt, hydrochloride salt, the hydrobromide salt, the nitrate salt, perchlorate salt, phosphate salt, sulfate salt, formate salt, acetate salt, aconite salt, ascorbate salt, benzosulphonate salt, benzoate salt, cinnamate salt, citrate salt, embonate salt, enantate salt, fumarate salt, glutamate salt, glycolate salt, lactate salt, maleate salt, malonate salt, mandelate salt, methane sulphonate salt, myristate salt, octanoate salt, phthalate salt, salicylate salt, sorbate salt, stearate salt, succinate salt, succinate dihydrate salt, tartrate salt, and the like. Such salts can be obtained following procedures known in the art.

The dopamine receptor agonist used for the purpose of the present disclosure can in various aspects be apomorphine, pramipexole, bromocriptine, cabergoline, ropinirole, or rotigotine, or a combination thereof.

The A2a antagonist can be administered concomitantly with the dopamine precursor or dopamine receptor agonist. By concomitant administration, it is intended that any form of administration can be used that enables the potentiation of dopamine precursor-induced treatment of the symptoms associated with a neurodegenerative condition. Such concomitant administration can include any form of administration in which 4-hydroxy-4-methyl-piperidine-1-carboxylic acid-(4-methoxy-7-morpholin-4-yl-benzothiazol-2-yl)-amide and/or dopamine precursors are administered together, such as in association in a pharmaceutical composition, or separately. A separate administration of A2a antagonist and dopamine precursor or a dopamine receptor agonist is preferably performed within a time frame that enables each of these compounds or a combination of these compounds to enter into blood circulation, pass through the hematoencephalic barrier, and exert its/their action on the brain, where the action of one compound potentiates the action of the other compound(s). The concomitant administration of least one of dehydroepiandrosterone and dehydroepiandrosterone-sulfate with a therapeutically effective amount of a dopamine precursor can be performed by any administration route known in the prior art and includes, but is not limited to, intravenous, subcutaneous, intradermal, transdermal, intraperitoneal, oral, parenteral, rectal, buccal, sublingual, and topical administration.

### Uses and Administration

The A2a antagonists and their pharmaceutically acceptable salts, optionally with dopamine precursors or dopamine receptor agonists can be used as medicaments, e.g., in the form of pharmaceutical preparations. The pharmaceutical preparations can be administered orally, e.g., in the form of tablets, coated tablets, dragees, hard and soft gelatine capsules, solutions, emulsions or suspensions. The administration can, however, also be effected rectally, e.g., in the form of suppositories, parenterally, e.g., in the form of injection solutions.

The compounds for use in the treatment can be processed with pharmaceutically inert, inorganic or organic carriers for the production of pharmaceutical preparations. Lactose, corn starch or derivatives thereof, talc, stearic acids or its salts and the like can be used, for example, as such carriers for tablets, coated tablets, dragees and hard gelatine capsules. Suitable carriers for soft gelatine capsules are, for example, vegetable oils, waxes, fats, semi-solid and liquid polyols, and the like. Depending on the nature of the active substance, carriers may or may not be required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols, and the like.

The pharmaceutical preparations can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing the compounds or a pharmaceutically acceptable salt thereof and a therapeutically inert carrier are also an object of the present disclosure, as is a process for their production, which comprises bringing one or more of the compounds and/or pharmaceutically acceptable acid addition salts and, if desired, one or more other therapeutically valuable substances into a galenical administration form together with one or more therapeutically inert carriers.

In accordance with the present disclosure compounds that are A2a antagonists as well as their pharmaceutically acceptable salts are useful in the control or prevention of illnesses based on the adenosine receptor antagonistic activity, such as Alzheimer's disease, Parkinson's disease, schizophrenia, neuroprotection, anxiety, pain, respiration deficits, depression, asthma, allergic responses, hypoxia, ischaemia, seizure, substance abuse, sleep disorders and cognition disorders. Furthermore, compounds of the present disclosure can be useful as sedatives, muscle relaxants, antipsychotics, antiepileptics, anticonvulsants, and cardioprotective agents and for the production of corresponding medicaments.

In certain aspects, the present disclosure relates to the treatment of disorders of the central nervous system, for example the treatment or prevention of certain depressive disorders, neuroprotection, Parkinson's disease, Alzheimer's disease, sleep disorders, cognitive impairment, and motor disorders.

The dosage can vary within wide limits and will, of course, have to be adjusted to the individual requirements in each particular case. In the case of oral administration the dosage for adults can vary from about 0.01 mg to about 1000 mg per day of an A2a antagonist compound or of the corresponding amount of a pharmaceutically acceptable salt thereof. The daily dosage can be administered as single dose or in divided doses and, in addition, the upper limit can also be exceeded when this is found to be indicated.

### Cognition Enhancement in Neurodegenative Diseases

In one aspect of the present disclosure, methods of treating a subject are provided wherein an A2a antagonist, or salts or solvates thereof is administered in combination with a dopamine precursor or a dopamine receptor agonist for a sufficient period of time necessary for treatment.

Enhancement of cognition occurs when changes in the damaged region neural function lead to changes in behavior or in the capacity for behavior.

An improvement in cognition of the patient suffering from a neurodegenerative disease can be assessed, for example, by using functional/behavioral tests to assess sensorimotor and reflex function of the patient's motor skills, such as posture, balance, grasp, or gait; cognitive skills; speech; and/or sensory perception including visual ability, taste, olfaction, and proprioception improve as a result of practicing the methods and compositions according to the present disclosure.

The patients treated with the methods and compositions of the present disclosure can be tested for enhancement of cognition. Examples of tests for measuring cognition include the following: Brief Psychiatric Rating Scale, Clinical Global Impression, Positive and Negative Symptoms Scale, Scale for Assessing Negative Symptoms, Young Mania Rating Scale, Cognitive subscale of the Alzheimer's Disease Assessment Scale, Clinician's Interview Based Impression of Change, Short Portable Mental Status Questionnaire, Folstein Mini-Mental Status Examination, Clinical Dementia Rating scale, Cambridge Neuropsychological Test Automated Battery, Wisconsin Card Sort Test, N-back working memory test, Weather prediction probabilistic learning test, Repeatable Battery for Assessment of Neuropsychological Status, or Continuous Performance Test Vigillance.

Optionally, brain activity can also be evaluated concurrently using a neuroimaging tool such as functional magnetic resonance imaging (fMRI), or a PET scan using a radiopharmaceutical such as [¹⁸F]fluorodopa or any other commercial single photon emission computed tomography (SPECT) ligand intended for Parkinson's disease in order to provide the dopaminergic status of the patient.

Continuous performance testing, in various forms, has become a standard clinical procedure. Generally speaking, continuous performance testing evaluates a subject's visual attention by displaying a series of visual stimuli, to which the subject is instructed to respond. In a typical case, often referred to as a "Go-No Go" test, the stimuli are of two types (the "Go" and "No Go" stimuli); the subject is instructed to respond only to the "Go" stimulus, and not to respond or "pass" when presented with the "No Go" stimulus. Data collected for each stimulus presented consists of the type of the stimulus; whether or not the subjects responded; and, if so, how long they took to respond. The continuous performance test has been in use since the 1950s.

The compositions of the present disclosure can be provided for use in one or more procedures. For treatment with a pharmaceutical composition comprising an A2a antagonist and a dopamine precursor or a dopamine receptor agonist for use in enhancing the symptoms of cognitive impairment, the compositions of the present disclosure can be provided as kits for use in one or more doses. The kits include a composition comprising the agent either as concentrates (including lyophilized compositions), which can be further diluted prior to use or they can be provided at the concentration of use, where the vials can include one or more dosages. Conveniently, in the kits single dosages can be provided in sterile vials so that the physician can employ the vials directly, where the vials will have the desired amount and concentration of agents. When the vials contain the formulation for direct use, usually there will be no need for other reagents for use with the method. The kits also can be in the form of a transdermal or transmucosal system for single or multiple applications. The subject compositions can be contained in packaging material, which comprises a label indicating that the subject compositions can be used to treat cognitive disorders in humans.

### EXAMPLES

Below are examples of specific embodiments for carrying out the present disclosure. The examples are offered for illustrative purposes only, and are not intended to limit the scope of the present disclosure in any way. Efforts have been made to ensure accuracy with respect to numbers used (e.g., amounts, temperatures, etc.), but some experimental error and deviation should, of course, be allowed for.

### EXAMPLE 1

### Treatment of Parkinson's Disease Patients Using a Combination of an A2a Antagonist and a Dopamine Precursor

Parkinson's disease (PD) patients participated in a double-blind, crossover study to assess the effect of co-administration of an A2a antagonist with a dopamine precursor on cognition and motor function. The A2a antagonist was 4-hydroxy-4-methyl-piperidine-1-carboxylic acid-(4-methoxy-7-morpholin-4-yl-benzothiazol-2-yl)-amide and the dopamine precursor was levodopa.

The patients were randomly allocated to 1 week of 4-hydroxy-4-methylpiperidine-1-carboxylic acid-(4-methoxy-7-morpholin-4-yl-benzothiazol-2-yl)-amide and levodopa, 1 week washout (levodopa only), then 1 week placebo plus levodopa or the reverse order. Optimal levodopa doses were established for each patient according to normal clinical practice. Active drug was administered at 60mg bid (N = 14) or 20mg bid (N = 12) of 4-hydroxy-4-methyl-piperidine-1-carboxylic acid-(4-methoxy-7-morpholin-4-yl-benzothiazol-2-yl)-amide. At the end of each treatment week, participants completed motor assessments, a Continuous Performance Test (CPT), a Go/No-Go (GNG) Test, and a 2-Back Test after overnight withdrawal of levodopa and again after intravenous (IV) infusion of a suboptimal level of levodopa.

The patients treated with 4-hydroxy-4-methyl-piperidine-1-carboxylic acid-(4-methoxy-7-morpholin-4-yl-benzothiazol-2-yl)-amide and levodopa demonstrated faster GNG reaction times (60 mg bid: p < 0.01; 20 mg bid: p = 0.05), without reduced accuracy (ps > 0.25) (**Figure 1**). **Figure 1** shows the mean change in reaction time (mS) from pre- to post-L-Dopa therapy (±SE). Subjects monitored a visual display while single uppercase letters were presented one at a time interspersed with the number "5". Participants were instructed to push a target response button at the occurrence of every letter (i.e., a "Go" response) but to withhold a response for the number 5 (i.e., a "No-Go" response). Target frequency was manipulated in a blocked fashion, with two levels of target frequency (83% letters: 17% "5"s; 50% letters: 50% "5"s).

There were no significant effects of either dose of 4-hydroxy-4-methyl-piperidine-1-carboxylic acid-(4-methoxy-7-morpholin-4-yl-benzothiazol-2-yl)-amide and levodopa on the 2-Back or CPT tests (all p-values > 0.05). Participants reported less sleepiness with 4-hydroxy-4-methyl-piperidine-1-carboxylic acid-(4-methoxy-7-morpholin-4-yl-benzothiazol-2-yl)-amide 60 mg bid, before and after levodopa (p < 0.05), compared to placebo, but no significant difference with 4-hydroxy-4-methyl-piperidine-1-carboxylic acid-(4-methoxy-7-morpholin-4-yl-benzothiazol-2-yl)-amide 20 mg bid (p > 0.05). There was a significant motor benefit of 4-hydroxy-4-methyl-piperidine-1-carboxylic acid-(4-methoxy-7-morpholin-4-yl-benzothiazol-2-yl)-amide 60 mg bid, before and after levodopa (p ≤ 0.05), on tapping speed and a significant 4-hydroxy-4-methyl-piperidine-1-carboxylic acid-(4-methoxy-7 morpholin-4-yl-benzothiazol-2-yl)-amide 60 mg bid and levodopa effect on the rapidly alternating hand movements item from the UPDRS (p = 0.03).

These results are consistent with a dose response curve for the beneficial interaction between A2a antagonists and levodopa on movement and cognition in PD. Thus, A2a antagonists and dopamine precursors can be used to enhance cognition and motor function in patients suffering from neurodegenerative diseases.

## Claims

1. 4-hydroxy-4-methyl-piperidine-1-carboxylic acid-(4-methoxy-7-morpholin-4-yl-benzothiazol-2-yl)-amide for use in the treatment of sleepiness associated with Parkinson's disease (PD) in a patient, wherein 4-hydroxy-4-methyl-piperidine-1-carboxylic acid-(4-methoxy-7-morpholin-4-yl-benzothiazol-2-yl)-amide is for administration in combination with levodopa, wherein
(a) the patient receives an optimal levodopa dose according to normal clinical practice that has been established by titrating the plasma level of levodopa to prevent a return of rigidity, tremor, and bradykinesia and to avoid dyskinesia; and
(b) 4-hydroxy-4-methyl-piperidine-1-carboxylic acid-(4-methoxy-7-morpholin-4-yl-benzothiazol-2-yl)-amide is administered to the patient at a dose of 60 mg twice a day.

2. 4-hydroxy-4-methyl-piperidine-1-carboxylic acid-(4-methoxy-7-morpholin-4-yl-benzothiazol-2-yl)-amide for use according to claim 1, wherein 4-hydroxy-4-methyl-piperidine-1-carboxylic acid-(4-methoxy-7-morpholin-4-yl-benzothiazol-2-yl)-amide is administered concomitantly or separately with levodopa.

## Patentansprüche

1. 4-Hydroxy-4-methylpiperidin-l-carbonsäure-(4-methoxy-7-morpholin-4-ylbenzothiazol-2-yl)amid zur Verwendung bei der Behandlung von mit Parkinson-Krankheit (PD) assoziierter Schläfrigkeit in einem Patienten, wobei das 4-Hydroxy-4-methylpiperidin-l-carbonsäure-(4-methoxy-7-morpholin-4-ylbenzothiazol-2-yl)amid für die Verabreichung in Kombination mit Levodopa bestimmt ist, wobei
(a) der Patient eine gemäß normaler klinischer Praxis optimale Levodopa-Dosis erhält, die durch Titrieren des Plasmaspiegels von Levodopa etabliert wurde, um ein Wiederauftreten von Rigidität, Tremor und Bradykinese zu verhindern und Dyskinesie zu vermeiden, und
(b) 4-Hydroxy-4-methylpiperidin-l-carbonsäure-(4-methoxy-7-morpholin-4-ylbenzothiazol-2-yl)amid dem Patienten zweimal täglich in einer Dosis von 60 mg verabreicht wird.

2. 4-Hydroxy-4-methylpiperidin-l-carbonsäure-(4-methoxy-7-morpholin-4-ylbenzothiazol-2-yl)amid zur Verwendung nach Anspruch 1, wobei 4-Hydroxy-4-methylpiperidin-1-carbonsäure-(4-methoxy-7-morpholin-4-ylbenzothiazol-2-yl)amid gleichzeitig mit oder getrennt von Levodopa verabreicht wird.

## Revendications

1. (4-méthoxy-7-morpholin-4-yl-benzothiazol-2-yl)-amide de l'acide 4-hydroxy-4-méthyl-pipéridine-1-carboxylique pour une utilisation dans le traitement de la somnolence associée à la maladie de Parkinson (MP) chez un patient, le (4-méthoxy-7-morpholin-4-yl-benzothiazol-2-yl)-amide de l'acide 4-hydroxy-4-méthyl-pipéridine-1-carboxylique étant destiné à une administration en combinaison avec la lévodopa,
(a) le patient recevant une dose optimale de lévodopa selon une pratique clinique normale qui a été établie par titration du niveau plasmatique de lévodopa pour empêcher un retour de rigidité, de tremblements, et de bradykinésie et pour éviter une dyskinésie ; et
(b) le (4-méthoxy-7-morpholin-4-yl-benzothiazol-2-yl)-amide de l'acide 4-hydroxy-4-méthyl-pipéridine-1-carboxylique étant administré au patient à raison d'une dose de 60 mg deux fois par jour.

2. (4-méthoxy-7-morpholin-4-yl-benzothiazol-2-yl)-amide de l'acide 4-hydroxy-4-méthyl-pipéridine-1-carboxylique pour une utilisation selon la revendication 1, le (4-méthoxy-7-morpholin-4-yl-benzothiazol-2-yl)-amide de l'acide 4-hydroxy-4-méthyl-pipéridine-1-carboxylique étant administré de manière concomitante ou séparément avec la lévodopa.
